# EUROPEAN PATENT SPECIFICATION

(11) **EP 3 116 433 B1**
(45) Date of publication and mention of the grant of the patent: **06.11.2019**
(21) Application number: 15718372.4
(22) Date of filing: 16.03.2015
(51) Int. Cl.: A61B 34/00, A61B 90/00, A61B 17/16, A61B 17/54, A61C 1/08

(54) **IMPROVEMENTS IN OR RELATING TO MEDICAL AND VETERINARIAN ROTARY TOOLS**
VERBESSERUNGEN AN ODER IM ZUSAMMENHANG MIT MEDIZINISCHEN ODER TIERMEDIZINISCHEN DREHWERKZEUGEN
AMÉLIORATIONS ASSOCIÉES À DES OUTILS ROTATIFS UTILISÉS EN MÉDECINE HUMAINE ET VÉTÉRINAIRE

(30) Priority: 14.03.2014 GB 201404667; 26.08.2014 GB 201415085
(43) Date of publication of application: 18.01.2017
(73) Proprietor: Medical Device Treatment Limited, Brigthon, East Sussex BN1 9SB (GB)
(72) Inventor: THOMAS, Rolf Lewis, Worthing Sussex BN11 2HJ (GB)
(74) Representative: Humphrey-Evans, Edward John
(86) International application number: PCT/GB2015/000092
(87) International publication number: WO 2015/136237

(56) References cited:
- GB-A- 2 345 254
- JP-A- 2007 276 017
- US-A- 4 072 440
- US-A1- 2009 118 742
- US-A1- 2011 046 626

## Description

### Field of the Invention

The present invention relates to medical and veterinarian rotary tools. In particular, the present invention relates to a rotary tool for the taking of biopsies and drilling into animal tissues such as nail, claw, hoof, bone and cartilaginous matter. More particularly, the present invention relates to nail care devices to improve the condition of nails, especially relating to the treatment of fungal infections.

### Background to the Invention

Onychomycosis is a fungal infection that causes the toenails and/or fingernails to thicken, discolour, and split. The prevalence of onychomycosis in the United States population as a whole is 13%, onychomycosis being more prevalent in the elderly (60%). Onychomycosis can result in permanent nail deformity. The disease can have a significant impact on a patient's quality of life arising from psychosocial issues e.g., due to concern regarding the appearance of one's toenails and fingernails, limiting a choice of sandals or shoes, on the one hand and pain associated with wearing shoes, walking and sports activities on the other hand. There may be occurrences of a foul odour.

Common causes of onychomycosis include dermatophytes, *Candida,* and nondermatophytic moulds. Dermatophytes are the fungi most commonly responsible for onychomycosis in the temperate western countries; while *Candida* and nondermatophyte moulds are more frequently involved in the tropics and subtropics with a hot and humid climate. Onychomycosis is more likely in the elderly due to diminished blood circulation, longer exposure to fungi, and nails which grow more slowly and thicken, increasing susceptibility to infection. Nail fungus tends to affect men more often than women, and is associated with a family history of this infection. Other risk factors include perspiring heavily, being in a humid or moist environment, psoriasis, wearing socks and shoes that hinder ventilation and do not absorb perspiration, going barefoot in damp public places such as swimming pools, gyms and shower rooms, having athlete's foot (tinea pedis), minor skin or nail injury, damaged nail or other infection, and having diabetes, circulation problems - which typically also lead to lower peripheral temperatures on hands and feet - or a weakened immune system.

In view of the above, it is important that diagnosis is correctly performed. To avoid misdiagnosis as nail psoriasis, lichen planus, contact dermatitis, nail bed tumours (melanoma), trauma, or yellow nail syndrome, laboratory confirmation may be necessary. The three main approaches are potassium hydroxide smear, culture and histology. This involves microscopic examination and culture of nail scrapings or clippings. Recent results indicate the most sensitive diagnostic approaches are direct smear combined with histological examination, and nail plate biopsy using periodic acid-Schiff stain. To reliably identify nondermatophyte moulds, several samples may be necessary. Clippings may have been treated with an anti-fungal agent and so may not provide an accurate diagnosis. Other nail conditions include subungual haematoma, usually caused by a crush injury, which causes symptoms such as intense pain and throbbing as blood collects under the nail. The nail develops a black discoloration overlying the nail bed but under the nail plate.

Treatment of onychomycosis is challenging because the infection is embedded within the nail and is difficult to reach. It may take a year or more of treatment, since new nail growth must entirely replace old, infected growth. Most treatments are either topical or oral antifungal medications. Oral medications include: terbinafine (76% effective), itraconazole (60% effective) and fluconazole (48% effective). Oral terbinafine is better tolerated than itraconazole. The use of an oral antifungal therapy in persons without a confirmed infection is a particular concern because of the side effects of that treatment. For superficial white onychomycosis, systemic rather than topical antifungal therapy is advised. Topical agents include: ciclopirox nail paint, clotrimazole, amorolfine or butenafine. Topical treatments need to be applied daily for prolonged periods (at least one year). Topical ciclopirox results in a cure in 6 to 9% of cases and it is believed that amorolfine might be more effective, although ciclopirox, when used with terbinafine, appears to be better than each agent alone.

A nail is homologous to a claw, which is a curved, pointed appendage, found at the end of a toe or finger in most amniotes (mammals, reptiles, birds). Nails and claws are made of hard protein called keratin. Claws are used to catch and hold prey in carnivorous mammals such as cats and dogs, but may also be used for such purposes as digging, climbing trees, self-defence, and grooming, in those and other species. In tetrapods, claws are made of keratin and consist of two layers. All Carnivora have claws, which vary considerably in length and shape. A nail that is big enough to bear weight is called a "hoof". Infections such as fungal growth issues affecting humans can also affect pets, farm animals etc.

Accordingly, treatment will typically involve nail removal by abrasive means and/or by drilling means. US4,990,134 (Auth) describes an abrasive tipped rotating cutting tool for use in removing abnormal deposits within a patient's vascular channels. The tip is covered with a material such as diamond grit, and is described as being rotated at high speed to pulverize any abnormal deposits contained within the vessel. The tip can be ellipsoidal in shape, and the coarseness of the abrasive material on the tip is variable from a particularly coarse grade, adjacent a distal end of the tip, to fine grade, adjacent a portion of the tip which has the widest diameter. The tool is described as using either an atraumatic tip or a preformable guide wire to guide the cutting tip through a patient's vessel.

US4,180,058 (Brem) describes a method for treating pathological conditions of the nail, particularly onychomycosis. The method comprises the breaching of the protective keratin of the nail to form an opening therein, placing a caustic-keratolytic agent in the opening to enlarge it, and treating the nail through the opening with topical therapeutic agents for the pathological condition being treated, for instance, an antifungal agent for onychomycosis. In addition, thick calluses and painful plantar warts are described as being removed with modifications of this method.

US6,572,580 (Feldman) describes a kit including a patch laden with topical anti-fungal medication and a set depth nail notcher for cutting a notch of predetermined depth in a nail plate, comprising a handle having a smooth flat surface, a longitudinal cutting edge protruding from the smooth flat surface to a height equal to the predetermined depth of the notch. A method is described for treating nail fungus, especially toenail fungus, comprising cutting a notch to a predetermined depth in a nail of a toe or finger infected with fungus and applying a topical anti-fungal medication to the toe or finger from a patch through the notch.

WO2006104600 (Jamison) describes a nail drill configured to create a hole in a nail, such as a fingernail or toenail. The nail drill has a shaft extending from a handle. A tip is disposed on an end of the shaft not coupled to the handle and has one or more cutting sections configured to cut into a nail. The nail drill is described as being utilized for relieving a pressurized area underneath a nail, such as bodily fluid that may have collected due to a prior trauma or other condition. The method for relieving the pressurized area includes positioning the tip onto an outer surface of the nail. The shaft may be rotated so that the tip turns and cuts a hole in the nail. The shaft is rotated until a hole is created through the nail, enabling the bodily fluid to pass from the pressurized area through the hole, thereby relieving the painful condition.

WO2013098555 (Thomas) provides a device including a hand held reusable electromechanical system and a single use cutting component as part of a treatment system for onychomycosis. The electromechanical system incorporates an electric motor and drive train to advance the cutting component through a nail, which also incorporates sensors for measuring the cutting resistance for the purpose of preventing the cutting device from overrunning into an associated nail bed below the nail. This document advises that once a suitable cutter has perforated a nail to produce an access port, a suitable anti-fungal agent can be applied to the nail bed through the access port. A helical cutter can be used to extract swarf, as is known and this document teaches that in a reverse mode of operation an antifungal agent may be applied, although scant detail is provided. US2014094808 (Herndon) teaches of a device comprising a bench-top drilling jig/assembly, a control module, mechanically connected to the drilling jig/assembly for controlling the depth of drilling by a drilling-motor and drill-bit; and a sensor, electrically connected to the drilling-motor and drill-bit and control module for detecting a change in an electrical impedance of a material being drilled. The drilling-motor and drill-bit is attached to a housing of the drilling assembly and is provided with an electrically insulated nosepiece made from e.g. Teflon having three or four feet to rigidly locate an entire drill assembly with respect to an outer surface of a material to be drilled. The system is requires hydration of nail material to be drilled, with the subject nail being soaked for two minutes in de-ionized water prior to commencement of drilling. A drill bit retaining chuck, is preferably of an electrically conductive material allowing for electrical impedance sensing through the drill motor to the drill bit in contact with the material to be drilled. US20140222003 (Herndon) relates to a precision bone drill and benefits from an electrically conducting nose piece comprising two tangs which engage into bone or nail substrate. US2009118742 (Medtronic Navigation Inc) teaches of a system for tracking a navigated instrument. JP2007276017 (FUKUDA R) teaches of a drill guide tool. GB2345254 (White, V) provides a drilling guide comprises a body made of moulded plastic forming a handle with through holes for locating a drill bit to prevent drill bit wander. US4072440 (Glover, R) provides a guide attachment for portable power drills.

### Object to the Invention

The present invention seeks to provide an improved mechanical system for use in the treatment of onychomycosis and other nail bed conditions. The present invention also seeks to provide easily replaceable components for the system.

### Statement of Invention

In accordance with a first aspect of the present invention, there is provided a nail and claw rotary tool that is operable to drill into hard nail and claw animal tissue substrate, the tool comprising a body, a chuck, a drill bit and a shroud, wherein; the body provides a mounting for the chuck to enable rotation of the chuck about an axis of rotation, the chuck being operably associated with a rotary input drive; the drill bit is an elongate member having a cylindrical shank at a first, proximal end, a cutting edge at a distal end; the chuck includes a circularly cylindrical aperture coaxial with said axis of rotation and is operable to accept a shank of the drill bit, there being a resilient member operable to assist coupling of the drill bit and the chuck, the shroud having a proximal coupling portion which engages with the body of the tool and a distal end which engages with the substrate the shroud defines an annular aperture tip about the axis of rotation, the aperture of the shroud having a diameter sufficient to allow axial movement of the distal end of the drill therethrough, the tip being operable to engage with the hard substrate; and, wherein the body is operable to permit axial movement of the chuck and drill bit relative to the shroud such that the drill bit can extend through the tip of the shroud, the shroud tip being operable to engage with a surface of the substrate. The present invention thus provides a hand-held device that can be employed with respect to a hard animal tissue substrate; the tip engaging with the substrate by way of friction or by way of physically engaging with a sharp edge or edges, whereby manual control can be effectively be performed without fear of slippage or damage arising from unwanted twisting and /or displacement arising from torque reaction upon start-up of the motor.

The shroud may comprise a foot arrangement having a non-invasive gripping surface, being provided with a rubber/polymeric plastics foot. In the alternative, the annular tip has a sharp or pointed edge, which engages with the substrate by cutting into the external surface of the substrate. The shroud tip can define a first section comprising a material operable to engage a surface of the substrate by friction and a second section operable to engage the surface by penetration, by way of a line contact, a point contact or a serrated contact. There can be four or more areas of differing surface engagement type. Applicants have determined that by engaging with the substrate and applying forces axially with respect to the drill bit, then the initial contact torque reaction can be resisted and by reason of the forces reacting to a foot or tip in direct contact with the substrate, then the difficulties encountered by prior art device which do not engage or otherwise contact with the substrate allow imprecise holes to be made, with a risk of damaging soft animal tissue. By arranging a control switch coaxially with respect to the drill-bit, it has been found that in the application of forces used to control the operation can assist in the drilling operation generally.

The shroud advantageously provides a passage for collection of swarf arising from drilling through the substrate. The present invention can thus provide a simple and easy to use device for producing holes in keratin for collecting biopsy material, drug delivery, relieving conditions such as subungal haematoma and the like. A specific advantage is that if a clinician decides to collect a biopsy for testing, then taking one from the nail root and below the nail plate (where the fungus resides) can be performed safely, simply and with minimal pain with the present invention. By having a shroud about the drill-bit, toward the shank, which opens out to an interior volume about the shank, then it has been found that swarf can be collected, for biopsy use later.

Conveniently, the drill bit comprises a composite member comprising a first elongate member having a primary shank at a first end of the elongate member and a cutting bit at an opposite end of the elongate member, wherein a secondary shank member is provided having a circular cylindrical symmetry about an axis and an elongate aperture coaxial within said axis, into which the primary shank can be inserted and fastened thereto, whereby to provide a composite drill bit with a shank diameter greater than a cutting diameter. By the provision of a larger diameter shank than would otherwise be the case, the drill bit is more easily handled; small drill bits are particularly brittle and can easily be broken by poor handling. The drill bits can be simply manufactured from HSS materials as is known and preferred in many situations; carbide drill bits and other types of drill can also be used to reduce cost and enable competitive pricing of disposable units. Packaging of such disposable units may contain features to make ejection and disposal of single use cutters simple and safe to perform and facilitate any subsequent processing by laboratories in the case of biopsy examinations.

The resilient member associated with the chuck and drill bit arrangement is a tight fit about or within one of the chuck and the drill bit and is operable to grip the other of the chuck and drill bit. Conveniently, the resilient member comprises a collet, associated with the shank of the drill bit, the collet having a resilient member which engages with the chuck. The collet can be manufactured from a machineable and mouldable plastics such as polypropylene. In one preferred embodiment, the resilient member comprises a collet arranged about a shank of the drill bit and has extending resilient fingers which operably clip into a surface feature of the chuck, to assist in at least one of retention of the drill bit and indication of securement of the drill bit. The resilient member can perform a first function to prevent the drill bit from sliding out of the chuck and a second function to prevent relative rotation thereof when in situ; this can be performed by elastic grip. In an alternative, mutually engagable portions can prevent relative rotation between the chuck and the shank of the drill bit.

Conveniently, the tool is arranged such that rotation of the chuck and drill bit is only possible after engagement of the tip of the shroud with a substrate, whereupon the drill bit is permitted to move outwardly of the tip, such that, the drill bit can engage with and drill into the substrate. Conveniently, the motor or power source is torque sensitive whereby to prevent unwanted soft tissue damage. Conveniently, power is supplied upon engagement of the shroud engaging a work-piece such that a pressure switch is activated. Conveniently, the rotation of the drill bit can rotate in first and second directions of rotation abut said axis. This can be beneficial not only in the collection of biopsy material, but can also assist in the delivery of medicament.

The shroud is provided with an apex that comprises an annular aperture tip with a substrate engagement portion; this aperture tip can comprise one of a sharp edge, a rough surface or an undulating surface. It is preferred that the shroud has have a sharp tip (relative to the keratin) and can be made from metals that will not easily corrode and are suited to sterilisation such as stainless steel or aluminium that has been anodised to increase hardness. The annular aperture tip can include a pressure-sensitive sensor, whereby to enable rotation upon engagement of the annular aperture tip with a surface. The shroud can be fabricated, for example, in part, close to the tip or generally, from a transparent or translucent material, whereby illumination from a light source within the shroud is possible or generally, to assist in viewing of the nail to be drilled. In the alternative, the tip, when provided with a foot having a non-slip polymeric plastics / rubber grip element can be provided in a number of styles. The foot may surround the annular opening for only 180° or 120°, for example, whereby to enable the tip to be placed close to the cuticle or eponychium of a fingernail / toenail. In a still further embodiment, the tip may define a plane that is not perpendicular to the axis of the drill bit, whereby the drill can enter a nail at an angle so as to enable a treatment, for example, to be applied under the cuticle or eponychium. Conveniently, the single use assembly prevents cross-infection and is sterilised during manufacture.

It will be appreciated that a diameter of the drill bit and a diameter of the annular aperture tip are selected such that, when extended through the aperture of the shroud, the drill bit is close fitting with respect to the aperture of the shroud, whereby to provide support and enable passage of swarf from a substrate and/or passage of a fluid to clean and/or passage of a medicament to the substrate. Conveniently, the shroud enables placement of medicament within the shroud for delivery of the medicament when a twist-drill bit is operated in reverse. Preferably, the shroud is provided with a light source, whereby to illuminate the hard animal tissue substrate to be drilled; the light source can be of a wavelength to provide germicidal irradiation, whereby to kill micro-organisms.

### Brief Description of the Figures

For a better understanding of the present invention, reference will now be made, by way of example only, to the Figures as shown in the accompanying drawing sheets, wherein:-
Figures 1 illustrate a known drilling system comprising a power tool, drill bit and depth gauge;
Figure 2 shows the tool of Figure 1 being operated by way of an articulated handling tool;
Figure 3 shows a second known drilling system with an electronic depth control system;
Figure 4 shows a first embodiment in accordance with the present invention in sectional view;
Figures 4a - 4k show specific details associated with the first embodiment;
Figure 5 shows an example of the present invention in operation;
Figures 6a - 6k show examples of the tip of a shroud in accordance with the present invention in sectional and perspective views;
Figure 7 shows a further view of a shroud tip in section;
Figure 8 shows another shroud in accordance with the invention;
Figures 9a - 9e show how a jig can be utilised in accordance with another aspect of the invention;
Figure 10 shows how a vacuum fan can be arranged; and,
Figures 11a & 11b show how debris can be collected.

### Detailed description of the Preferred Embodiments

There will now be described, by way of example only, the best mode contemplated by the inventor for carrying out the present invention. In the following description, numerous specific details are set out in order to provide a complete understanding to the present invention. It will be apparent to those skilled in the art, that the present invention may be put into practice with variations of the specific.

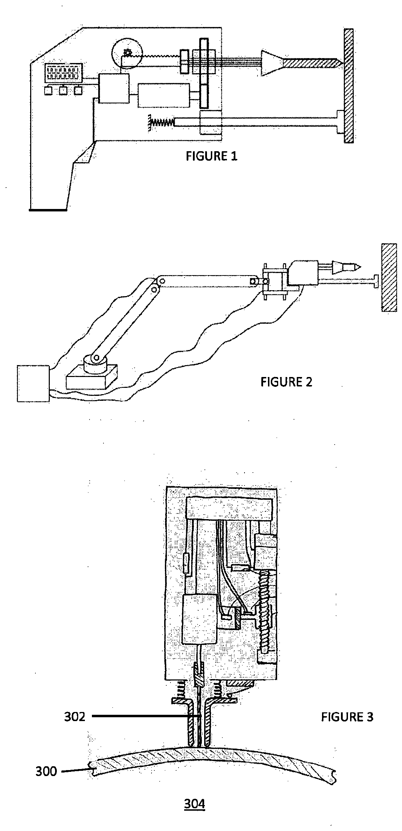

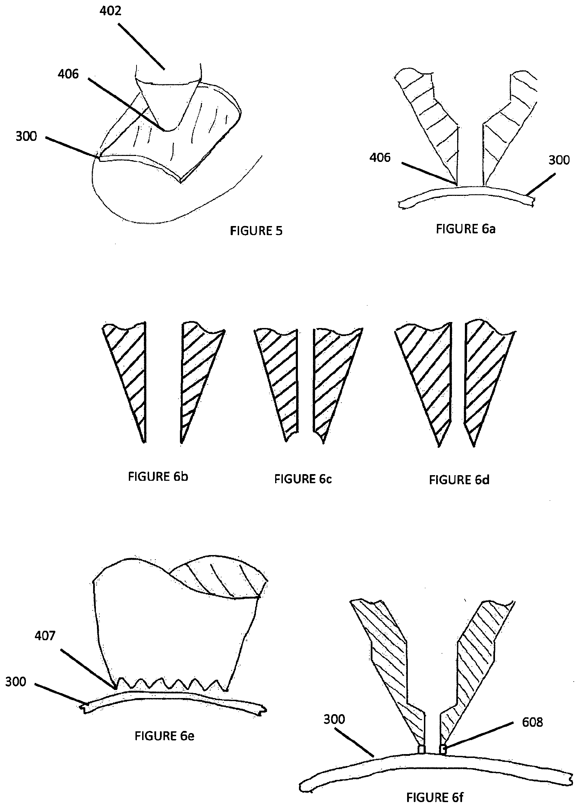

Referring to Figure 3, the present invention relates to a device that is operable to drill a hole through a nail 301 using a drill bit 302 in a controlled manner which device is arranged to prevent an overrun of the drill bit in use, having progressed through the nail, into the nail bed 304 lying below. This prior art device benefits from motor control but suffers from being hand-held: the device must be held in against the nail 300 whereby to determine differences in torque resistance to the drill bit as it rotates. In this known system a drill bit is fixedly attached to a drill bit holder (chuck) in a controlled manner in order to accurately track the progress of the drill bit 302 through the nail 300 thus preventing it overrunning into the nail bed. When the device is first placed against the nail 300 the cutter 302 will not be in contact with the nail 300 (see FIG 5). Upon activation the cutter will start to rotate and will then be advanced in an axial direction towards the nail 300. When the cutter 302 makes contact with the nail 300 there will be a reaction force from the nail 300 in a direction corresponding to the axis of the cutter 302 and a reaction torque will act with respect to the cutter 302. This prior teaching provides a motor control responsive to such forces.

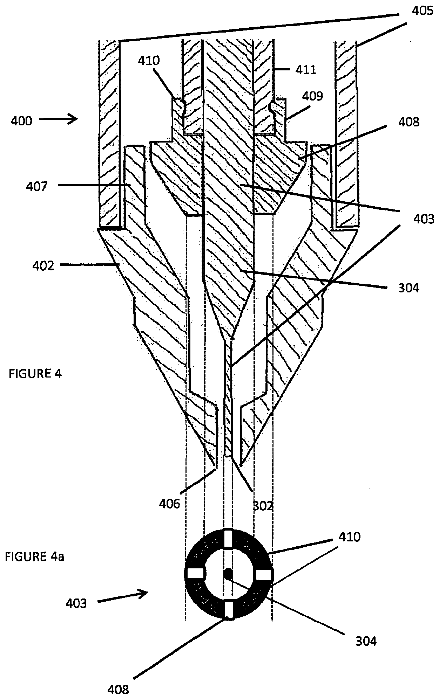

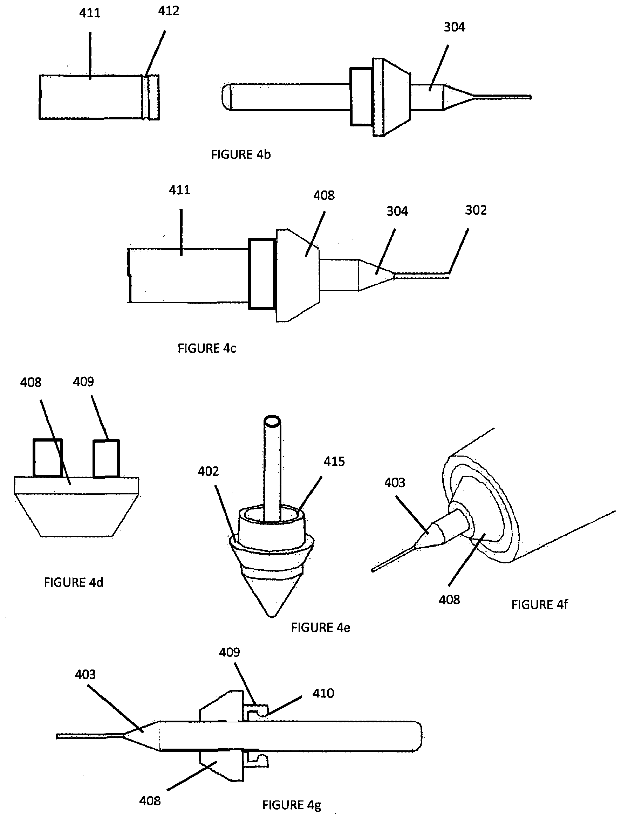

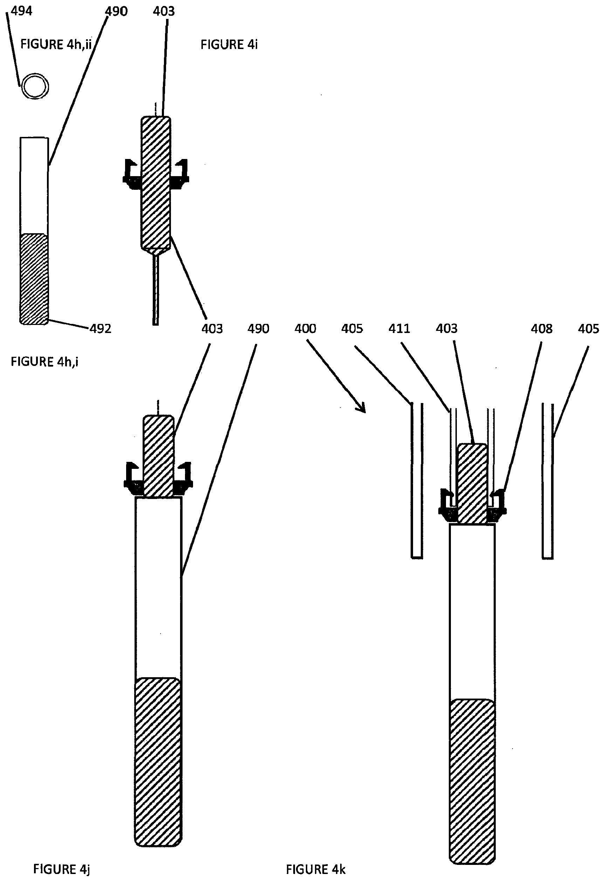

Notwithstanding the above, difficulties can arise, not least through the axis of cutting changing through relative movement between the drill bit and the nail 300. Accordingly, the present invention provides a number of modifications to the design to overcome or ameliorate such problems. Referring now to Figure 4, numeral 400 refers generally to a drilling assembly in accordance with one aspect of the invention. The drilling assembly can be coupled to a motor for rotational drive or may be coupled to an output coupling of a flexible shaft, wherein the motor provides rotational drive at an input coupling of the flexible shaft. The assembly comprises a composite drill 403 retained for rotation within a chuck which is surrounded by a shroud 402, which depends form an upper sleeve member associated with the motor drive unit (not shown), the composite drill being movable relative to the shroud. The shroud, by means of an annular sleeve 407 at a proximal portion of the shroud, engages with a corresponding mating face associated with the body 405 of the rotational tool. The composite drill 403 comprises a drill bit 302 and a shank element 304 (which receives rotational drive about axis Z from a rotational power from coupling member 411), associated with a control tool or device (not shown). For ease in handling and to assist in the coupling of the shank to the chuck, the outside diameter of the shank (typically, Φ = 3.175 mm, corresponding to 1/8th inch, a standard diameter for many engineering applications) is considerably greater than the drill bit (typically, Φ = 0.1 - 2mm), although this would not be necessary for larger drill bits. At a distal portion of the shroud 406, the shroud reduces in diameter to define an apex with an aperture through which the drill bit can pass and engage with a patient body part. For example, as shown in Figure 5, the apex abuts a nail plate 300 prior to the drill bit being brought towards and through the aperture 406 at the base of the shroud. The chuck coupling member 411 has an inside diameter of 3.175 mm, corresponding to the outside diameter of the shank and an outside diameter of 5.5 mm. Whilst a resilient clip is shown in the specific embodiment, the skilled man will realise that a magnetic assembly can retain ferromagnetic drill bits, as such magnetic connections are widely used many in non-percussive applications, such as multi-bit magnetic screw-driver assemblies.

In detail, and referring also to Figures 4 a - c, the shank 304 is retained within a power transfer chuck 411 that enables an extremely simple change of drill bit. With specific reference to Figure 4a, which shows a plan view of the composite drill 403 from the proximal end, although the drill bit cutter 302 is indicated in outline. The shank of the composite bit is provided with a plastics collet 408, which has four upstanding elements 409 with inherent resiliency, being manufactured from machinable and mouldable plastics such as polypropylene. Four inwardly extending features 410 are defined at the end of the upstanding elements 409. These inwardly extending features locate within an annular depression 412 associated with the transfer chuck 411, whereby not only to assist in the confirmation of the drill bit being fitted satisfactorily but to assist in gripping an outside surface of the power transfer chuck 411. An insertion tool operable to protect a brittle drill bit and reduce a risk of contamination can be provided, comprising an elongate tool with an inwardly direct opening operable to couple about the shank in a loose fit, an abut against the collet, whereby the drill bit is not touched and therefore not contaminated as the drill bit is placed within the transfer chuck 411.

The shank is machined to an outside diameter of 3.175 mm and the inside diameter of the chuck corresponds to provide a sliding fit, to prevent axial jitter of the drill in use; an air passage (not shown) is provided whereby to enable the shank to be simply inserted into the chuck. The tightness of fit is controlled by an amount of interference; the "allowance". Formulas are readily available to the skilled man t to compute this allowance (planned difference from nominal size) that will result in various strengths of fit such as loose fit, light interference fit, and interference fit. The value of the allowance depends on which material is being used, how big the parts are, and what degree of tightness is desired. Such values have already been worked out in the past for many standard applications, and they are available to engineers in the form of tables, obviating the need for re-derivation. Therefore, if a loose fit is desired for a 3.175 mm shaft made of a particular grade of stainless steel, the engineer can look up the needed allowance in a reference book or computer program, rather than using a formula to calculate it. A flattened face together with a corresponding projection on the shank (not shown) could also be provided, which together with a resilient element such as a clip or similar could enable secure retention of the composite bit within the chuck. Prior to use, like all medical instruments, the composite drill must be sterilised - although this system easily adapts to the disposable, single use environment, typical of modern surgeries/health care centres, the sterilisation conveniently taking place during manufacture, by sterilisation by moist heat, gamma irradiation, and other methods, whereby sterile packed products are provided at said manufacturing facility. The drill together with a plastics shroud can easily be provided as a kit of parts for single use with a particular patient, as shall be discussed below. This would be advantageous in that a single use shroud would assist with any collection of biopsy material, since the drill can be placed in a biopsy package, without being required to be cleaned, possibly with loss of material.

Figure 4d shows a perspective view of a collet 408, on its own; the four upstanding members 409 are clearly identifiable; the division of the otherwise circumferential body allowing flexing of the upstanding members outwardly. Figure 4e is a perspective view of the proximal end of the shroud and shows part of the chuck that, in use, is coupled with a type of device as shown in Figure 3 or be coupled to a flexible drive shaft, where a power tool arrangement is inconvenient, for example in veterinary applications. A flexible shaft can transmit rotary motion much like a solid shaft, but, it can be routed over, under, and around obstacles that would make using a solid shaft impractical. A flexible shaft assembly consists of a rotating shaft (sometimes called a core) with metal end fittings for attachment to mating parts. A protective outer casing is used when necessary. Notwithstanding this, torque reaction delay times may arise from motor stop to tool stop due to an inherent resilience in the drive shaft, which increases with increasing length of driveshaft. Figure 4f shows a composite drill 403 when in place in a power tool with the shroud removed. Figure 4g shows the drill assembly in outline, clearly showing the upstanding members 409 of the collet 408, with the inwardly directed features 410.

Figure 4h shows an example of an insertion tool 490 having a handle portion at a first end and a cylindrical aperture operable to enclose the shank of a drill bit assembly; Figure 4hi shows the tool in plan view; Figure 4hii shows the tool end on showing the a cylindrical bore. Figures 4i and Figure 4j show how the drill assembly is inserted into the tool, whereby handling and positioning is simplified. Figure 4k shows how the assembly engages the chuck of the tool once the shroud has been removed. If the tool is made of plastics, then it can assist in protection of the drill during storage and transport and be part of a single-use system. Further features to assist in the loading and ejection of the drill bit from the chuck can be provided: for example, the packaging could include the holder may be provided with alignment markings, to assist loading of a drill bit and secure removal therefrom. A further feature or component of the packaging could assist in the provision of a seal for biopsy and to prevent potential cross-contamination issues, although standard stock control techniques should be maintained at all times.

Figure 5 shows how the aperture 406 of the shroud 402 engages with, in this example, a fingernail 300, whereby to enable a secure, non-slip placement of the device and, therefore, reliable drilling of the nail. Figure 6a shows in cross-section how the annulus 406 abuts a nail plate 300, whereby to stabilise the drilling system. The drill bit can conveniently be made from a variety of tool steels, as is known. It is preferred that the shroud has have a sharp tip (relative to the keratin) and can be made from metals that will not easily corrode and are suited to sterilisation such as hardened steel, stainless steel or aluminium that has been anodised to increase hardness. The shroud either as a whole or just about the tip can be made from a transparent or translucent material, whereby illumination from a light source within the shroud is possible or generally, to assist in viewing of the nail to be drilled. The form of the shroud should allow good visibility to a work piece and access to all areas (access to the nail root is critical in the treatment of onychomycosis). The annular aperture tip can include a pressure-sensitive sensor, whereby to enable control of a power source to the tool upon engagement of the annular aperture tip with a surface. The annulus tip member could be fitted to a plastics shroud or an aluminium shroud, although, it will be realised a multitude of materials can be employed, it being realised that the components need to be autoclavable or otherwise sterilised. Conveniently, the single use assembly prevents cross-infection and is sterilised sterilised during manufacture.

Figures 6b - 6d show three different types of annulus in cross-section, which have sharp edges to enable an annulus to assist in engagement with a nail, to prevent slippage of one with respect to the other. The chuck is arranged so that it grips the collar 408 associated with the drill bit 302 by means of an interference fit. This provides a specific advantage in that, in the event the device that drills into the nail is knocked and the drill bit is forced off-axis with respect to a nail element, then the drill bit, in the event that it locks onto the nail rather than rotating with respect to the nail can slip within the chuck momentarily prior to the torque sensor preventing the motor from further rotation.

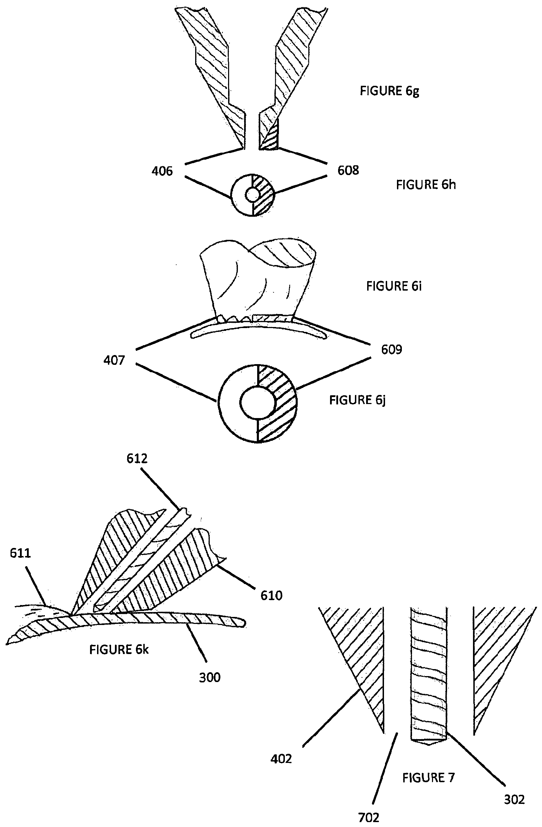

Figure 6f relates to a different form of shroud where alternatively or additionally a sharp substrate engaging tip is replaced with a rubber-like element that grips the surface. The rubber-like compound can be selected - though not necessarily restricted to materials made form or comprised of Suitable materials include, but may not be restricted to the following: a silicone rubber, a nitrile compound such as a hydrogenated nitrile compound, ethylene-propylene compounds, fluorocarbons, fluorosilicones, styrene-butadiene compounds, chloroprene compounds etc., the material being chosen because of its high-grip properties with respect to nail and nail-like substrates. Figure 6g shows a first alternative where the sharp substrate engaging tip is supplemented with a rubber-like compound. It will be appreciated that a rubber-like grip will not affect the surface of the nail in the same fashion as a sharp tip; it will be less painful in application of axially directed forces as the tool is used. The grip must be such that the torque reaction of the tool is taken into account and the nail is not damaged upon start-up of the tool. Specifically, with reference to Figure 6f, the shroud has a tip that comprises a rubber foot, which prevents slippage in normal use. Figure 6g shows a second further variation, where a tip has half rubber grip element 608 and half surface engaging tip 406. Figure 6h shows the overall view from underneath, with the tip 406 defining a sharp edge on an inside of the tip. In contrast, figure 6i shows a further shroud wherein the tip is half polymeric rubber foot and half a serrated tip 407: this time the serrated tip lies on the outside of the tip member; figure 6j shows how this appears form the underside. By having two types of surface grip/engagement means, the tool is quite adaptable to various conditions of tool. Sometimes the fungal nail will damage the substrate surface such that the entry of the surface of the nail substrate will actually be ineffective to a torque transfer upon start-up of the motor and so the use of a rubber part will assist in a satisfactory placement and maintenance in position of the tool which can therefore be most satisfactorily be held and retained by hand, enabling control by the clinician.

Figure 6k shows a still further variation, wherein the shroud 610 has a tip which lies in a plane that is not perpendicular to the axis of the drill 612. This enables a drill to penetrate the nail or other material at an angle other than normal to a surface thereof, whereby to avoid damaging the cuticle, for example in the case of a human nail.

A further significant advantage provided by this system is that new drill bits, of typically 1mm in diameter, can be replaced simply by removing the shroud - conveniently located with a rubber O-ring about a part of the body of the tool - inserting the shank into a cylindrical aperture defined within a cylindrical chuck, a resilient collet engaging with an outside surface of the chuck - although other methods of fastening can be easily envisaged. The skilled man will readily appreciate the convenience: there is no possibility of any complication - substantial or otherwise - encountered when chuck key or hand-grip fastening procedures need to be deployed. Instead, a simple push on fit is provided by the collet of the shank gripping the chuck. A chuck is a specialized type of clamp used to hold an object, usually an object with radial symmetry, especially a cylindrical object, and are used most commonly used to hold a rotating tool (such as the drill bit in a power tool). Many chucks have jaws, which can be arranged in a radially symmetrical pattern (like the points of a star) to hold a tool or work-piece, although this does not prevent axial run-out, which is not normally an issue in, say household diy drilling projects, but will obviously have a significant effect when drilling nail, bone etc. since the tolerance corresponds in magnitude to the run-out in such chucks. Often the jaws will be tightened or loosened with the help of a chuck key - a wrench-like tool, typically with a circumferential gear arrangement for engagement with a corresponding radial tightening ring about the chuck. Keyless chucks are also available, their tightening and loosening being performed by hand force alone. Additionally, by having a simple unitary design, the chuck can be manufactured from materials that are sterilisation compatible or deliberately non-compatible in order to prevent reuse.

Conveniently, single use components are provided with tools that assist in placement within a chuck, for example, in the provision of an alignment mechanism in association with the shroud. Such replaceable tools can comprise part of a treatment package whereby drill bits are provided together with receptacles to not only ensure safety in the provision of the tools but also to enable safe disposal, including the option of providing soiled drill bits and shank for biopsy examination. Conveniently, the shroud can be placed in a package for biopsy examination, in the event that the shroud enables collection of the swarf arising from drilling through a nail or other substrate.

By having a shank of the drill bits of specific lengths, then control of penetration though particularly deep nail can be assisted and/or the extent of penetration is limited. The diameter of the aperture defined by the annulus is also variable, commensurate with the size of drill bit as shall be discussed below. Figure 6e shows a still further embodiment wherein the annulus 407 comprises a jagged edge. This design ensures engagement with nail plate, surface gripping features, stability features, forms a seal between a nail plate and the annulus tip, with advantages that can be realised in biopsy capture and drug delivery.

Returning to Figure 4e, a perspective view of a distal end of a shroud is shown, which provides a volume 415 which can accommodate a number of features including, but not limited to, a vacuum system to assist in removal and possible storage of swarf form any associated drilling operations; storage for application of medicaments; placement of light sources etc.. Figure 7 shows the relative sizes of a drill bit 302 within the nozzle of the shroud 406, where an annular space is defined, which space can be used for biopsy collection, drug delivery, etc., although if the tolerance was tighter, the annulus could also act as a drill guide: the annular gap 702 can be critical for a particular function, given that if a drug is being delivered in reverse rotational mode i.e. acts as screw-feed for drug, which if supplied as a cream may have a viscosity which if delivered by any other means would not necessarily be present in the desired area by virtue of the presence of air-gaps. The use of free-flowing liquid medicaments means that medicament loss is great and therefore the overall treatment can be ineffective. The gap between the outside of the drill and the inside of the tube can be critical. Obviously, interfaces between relatively rotating parts are best provided with O-rings to prevent accumulation of unwanted debris, medicament or other products, or to provide a sealing feature, to ensure prevention of leakage of material; including but not restricted to biopsy material (to prevent cross-infection).

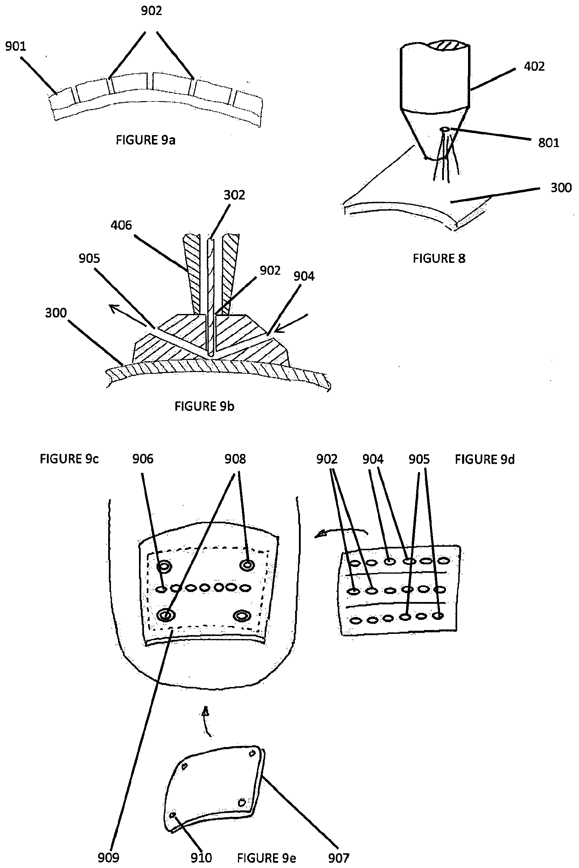

Figure 8 shows a still further variation, wherein the shroud 801 is provided with an aperture, which aperture can provide means for the delivery of drug and removal of debris by suction, or airflow towards nail. The present invention can overcome present problems encountered in the delivery of a drip- fed medicament as discussed above, by using the drill bit in reverse rotation, together with use of the as a feed for a medicament. A light source may be arranged within or about the shroud, to assist in illuminating the drilling area.

In accordance with a further aspect of the invention, there is provided a work-piece jig 901 which cooperates with the annular tip of the shroud 406 to ensure accurate placement of the drill bits using the rotary tool of the invention, under manual control with respect to a nail 300. Referring to Figure 9a, a section through the jig 901 shows guide channels 902 for use in enabling drill bits to accurately be placed with respect to the nail. Figure 9b shows the jig at 90° to jig 901 with respect to the view shown in Figure 9a, together with the tip of the shroud 406 engaging with the top surface of the jig. Not shown, but an apertured button top, can be arranged so that the tip can couple with the jig, to enable repeatable drilling, with the jig also being provided with conduits 904 and 905 for the introduction of air whereby to clear swarf, for example, or for the introduction of a medicament, with the conduit 905 for the removal of biopsy-swarf. Channels 904 and 905 are optional and can be placed independently of one another. The jig can be placed upon the nail surface; the use of glues across the whole of a nail surface is not recommended, but by the placement of locating dots 908 upon a nail surface, attached by a suitable glue, such as a cyanoacrylate glue, then the jig can be located with respect to the nail.

With reference to Figure 9c - the locating dots, retained by glue in confined areas at four points on the nail surface ensure that the jig is securely located, with the underside of the jig having corresponding locating means associated complementary to the locating dots. After the jig has been successfully been employed to enable the rotary to drill into the nail under the hand-held (manual) control of an operator, collect biopsy material and deliver medicament as appropriate, then a false nail 907, also with complementary locating dots 910 can be placed upon the nail, to protect and seal the area - to the level required for the particular condition of the nail. Other forms of false nail can be employed, with channels for the provision of medicament over time. It will be appreciated that different jigs and false nails having different widths and arcs of curvature will be required to accommodate the differing nature of human beings. It can be envisioned, without departing from the invention, that many variations are possible.

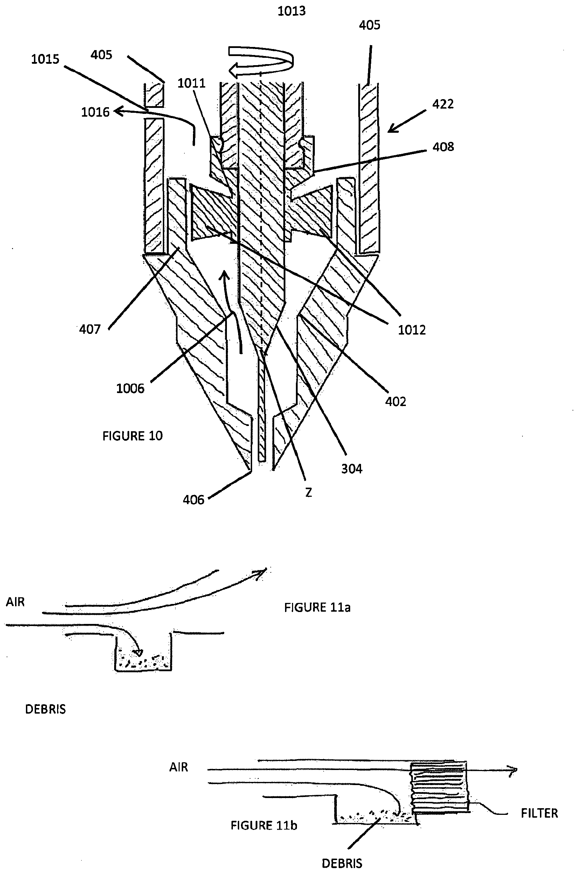

Figure 10 shows a further embodiment 422 wherein a fan assembly 1011 is present, placed a short axial distance from collar 408 comprises four blades 1012, arranged to provide a relative vacuum at the nozzle 406 upon rotation indicated by reference numeral 1013. Arrow 1006 indicates the direction the air and debris flow from the nozzle; Air indicated at 1016 exits the motor unit through aperture 1015 defined in the wall of the casing 405. The addition of a fan or fan blades to the rotating component generates an airflow whereby debris is removed from the drilling site. The fan may be included or separate to the clip used to retain the drill bit within the drive shaft. Conveniently, a tube is fastened to the aperture 1015 by way of a coupling member or otherwise whereby the air plus nail debris can be guided away.

Ideally, there is also provided a trap or other containment system, whereby the debris can be removed from the airflow and collected for biopsy use. The tube can lead to a collection trap. Figure 11a shows a simple trap whereby debris is deposited either by virtue of changing air velocity and the Bernoulli effect or upon the air flow hitting a baffle member. Preferably, and in accordance with the system shown in Figure 11b, a filter system is provided whereby the debris is collected on the inside face of the filter or drops down into a containment area. The trap is ideally easily removable to enable simple collection of the debris. By having the fan removed with the drill bit, single use of the fan ensures that cross contamination is reduced. Conveniently, an inner duct (not shown) is provided whereby the duct, tube and fan are removed together with the shroud 402; after use all are removed from the drill tool.

The rotary tool can also be provided with a window or is either transparent or translucent, whereby the hard animal tissue substrate to be drilled can be viewed through the shroud. Conveniently, the shroud is provided with a light source, whereby to illuminate the hard animal tissue substrate to be drilled. Preferably, the light source is a broad spectrum light source that can also provide germicidal ultra-violet (UV) light, whereby to provide irradiation treatment, in addition to the provision of a medicament. It will be appreciated that the effectiveness of germicidal ultra-violet light in such an environment depends on a number of certain factors: the length of time a microorganism is exposed to ultra-violet light, the presence of particles that can protect the micro-organisms from ultra-violet light, and a micro-organism's ability to withstand ultra-violet light during its exposure. Further, the effectiveness of this form of sterilization can be dependent upon line-of-sight exposure of the micro-organisms to the ultra-violet light; dirt from a debris-removal airflow upon a lamp can reduce effective light output, whilst poor lamp cooling under the airflow can also detrimentally affect ultra-violet light output. Accordingly, the light source can conveniently be placed such that impingement of the lamp with any airflow does not occur, for example by use of a shield for the lamp together with a parabolic reflector. It should also be recognised that in use, such bulbs require annual replacement and scheduled cleaning to ensure effectiveness. Conveniently, the window of the shroud or the transparent/ translucent material of the shroud is not transparent to the ultra-violet light.

As is known, per WO2013098555 in the name of the inventor of present invention, the drill system employs torque sensitive sensors whereby drilling is stopped after drilling through the nail has completed, prior to penetration into the nail bed. The system uses a drilling procedure that allows the motor to be started once the shroud has been securely located and a pressure switch has been activated. The forces can be measured using various sensors, for example.

Whilst the present invention has been described solely with reference to matters of onychomycosis in the nails of human beings, the ambit of overall uses is not so limited. The present invention can be used, for example, in the collection of biopsy matter, drug delivery, subungual haematoma, animal claws, skulls e.g. hydrocephalus.

## Claims

1. A nail and claw rotary tool for drilling into hard nail and claw animal tissue substrate, the tool (400) comprising a body (405), a chuck (411), a drill bit (302) and a shroud (402), wherein;
the body provides a mounting for the chuck to enable rotation of the chuck about an axis of rotation (Z), the chuck being operably associated with a rotary input drive;
the drill bit is an elongate member having a cylindrical shank at a first, proximal end, a cutting edge at a distal end;
the chuck includes a circularly cylindrical aperture coaxial with said axis of rotation and is operable to accept a shank (304) of the drill bit, **characterised in that** there is provided a resilient member (408) operable to assist coupling of the drill bit and the chuck,
the shroud (402) having a proximal coupling portion (407) which engages with the body of the tool and a distal end which defines an annular aperture tip (406) about the axis of rotation, the aperture of the shroud having a diameter sufficient to allow axial movement of the distal end of the drill therethrough, the tip being operable to engage with a substrate; and,
wherein the body is operable to permit axial movement of the chuck and drill bit relative to the shroud such that the drill bit can extend through the tip.

2. A rotary tool for drilling into hard nail and claw animal tissue substrate according to claim 1, wherein the tip of the shroud (406) is operable to engage with the substrate by way of friction, the tip being provided with a rubber or rubber-/polymeric- like compound.

3. A rotary tool for drilling into hard nail and claw animal tissue substrate according to claim 1, wherein the tip of the shroud is operable to physically engage with the substrate by way of the tip entering into the surface of the substrate , the tip defining one of a line contact, a point contact or a serrated contact.

4. A rotary tool for drilling into hard nail and claw animal tissue substrate according to claim 1, wherein the tip defines a first section comprising a material operable to engage a surface of the substrate by friction and a second section operable to engage the surface by penetration, by way of a line contact, a point contact or a serrated contact.

5. A rotary tool for drilling into hard nail and claw animal tissue substrate according to claim 1, wherein the drill bit comprises a composite member comprising a first elongate member having a primary shank (403) at a first end of the elongate member and a cutting bit (302) at an opposite end of the elongate member, wherein a secondary shank member is provided having a circular cylindrical symmetry about an axis and an elongate aperture coaxial within said axis, into which the primary shank can be inserted and fastened thereto, whereby to provide a composite drill bit with a shank diameter greater than a cutting diameter.

6. A rotary tool for drilling into hard animal tissue substrate according to any one of claims 1 - 5, wherein the resilient member (408) is a tight fit about or within one of the chuck and the drill bit and is operable to grip the other of the chuck and drill bit.

7. A rotary tool for drilling into hard animal tissue substrate according to any one of claims 1 - 6, wherein the resilient member (408) comprises a collet, associated with the shank of the drill bit, the collet having a resiliency whereby to operably engage with the chuck.

8. A rotary tool for drilling into hard animal tissue substrate according to claim 7, wherein the resilient member (408) comprises a collet arranged about a shank of the drill bit and has extending resilient fingers which extending resilient fingers are operable to clip into a surface feature of the chuck, whereby to assist in at least one of i) retention of the drill bit and, ii) indication of securement of the drill bit.

9. A rotary tool for drilling into hard animal tissue substrate according to any one of claims 1 - 8, wherein the tool is arranged such that rotation of the chuck and drill bit can occur only after engagement of the tip with a substrate, whereupon the drill bit is permitted to move outwardly of the tip, such that, the drill bit can engage with and drill into the substrate.

10. A rotary tool for drilling into hard animal tissue substrate according to any one of claims 1 - 9, wherein a torque sensitive motor is operable to rotatably drive the drill bit and chuck.

11. A rotary tool for drilling into hard animal tissue substrate according to any one of claims 1 - 10, wherein the motor is operable in first and second directions of rotation about said axis.

12. A rotary tool for drilling into hard animal tissue substrate according to any one of claims 1 - 11, wherein the shroud apex provides an annular aperture tip with a substrate engagement portion.

13. A rotary tool for drilling into hard animal tissue substrate according to any one of claims 1 - 12, wherein the aperture tip is annular and includes a pressure-sensitive sensor, whereby to enable rotation upon engagement of the annular aperture tip with a surface.

14. A rotary tool for drilling into hard animal tissue substrate according to any one of claims 1 - 13, wherein the diameter of the drill bit and a diameter of the annular aperture tip are selected such that, when extended through the aperture of the shroud, the drill bit is close fitting with respect to the aperture of the shroud, whereby to provide support and enable passage of swarf from a substrate and/or passage of a fluid to clean and/or passage of a medicament to the substrate.

15. A rotary tool for drilling into hard animal tissue substrate according to any one of claims 1 - 14, wherein the shroud enables collection of swarf from a substrate for biopsy purposes or otherwise.

## Patentansprüche

1. Ein Nagel- und Klauen-Drehwerkzeug zum Bohren in ein hartes tierisches Nagel- und Klauen-Gewebesubstrat, das Werkzeug (400) umfasst ein Gehäuse (405), eine Spannvorrichtung (411), einen Bohreinsatz (302) und eine Ummantelung (402), wobei;
das Gehäuse eine Befestigung für die Spannvorrichtung bietet, um die Rotation der Spannvorrichtung um eine Rotationsachse (Z) zu ermöglichen, die Spannvorrichtung ist funktionsbereit mit einem rotierenden Eingangsantrieb verbunden;
der Bohreinsatz ist ein längliches Teil mit einem zylindrischen Schaft an einem ersten, proximalen Ende, und einer Schnittkante an einem distalen Ende;
die Spannvorrichtung umfasst eine kreisförmige zylindrische Öffnung, die koaxial zu der besagten Rotationsachse verläuft und funktionsbereit ist, um einen Schaft (304) des Bohreinsatzes aufzunehmen, **gekennzeichnet dadurch, dass** ein elastisches Teil (408) bereitgestellt wird, das funktionsbereit ist, um das Koppeln des Bohreinsatzes und der Spannvorrichtung zu unterstützen, die Ummantelung (402) hat ein proximales Kopplungsteil (407), das sich mit dem Gehäuse des Werkzeugs verbindet, und ein distales Ende, das eine ringförmige Öffnungsspitze (406) um die Rotationsachse definiert, die Öffnung der Ummantelung hat einen Durchmesser, der ausreicht, um die axiale Bewegung des distalen Endes der Bohrung dort hindurch zu erlauben, die Spitze ist funktionsbereit, sich mit dem Substrat zu verbinden; und,
wobei das Gehäuse funktionsbereit ist, eine axiale Bewegung der Spannvorrichtung und des Bohreinsatzes im Verhältnis zur Ummantelung zu erlauben, so dass sich der Bohreinsatz durch die Spitze erstrecken kann.

2. Ein Drehwerkzeug für das Bohren in ein tierisches Nagel- und Klauen-Gewebesubstrat nach Anspruch 1, wobei die Spitze der Ummantelung (406) funktionsbereit ist, um sich mit dem Substrat durch Reibung zu verbinden, die Spitze ist mit einem Gummi oder einer gummi-/polymerähnlichen Verbindung ausgestattet.

3. Ein Drehwerkzeug für das Bohren in ein tierisches Nagel- und Klauen-Gewebesubstrat nach Anspruch 1, wobei die Spitze der Ummantelung funktionsbereit ist, um sich physisch mit dem Substrat zu verbinden, indem die Spitze in die Oberfläche des Substrats eindringt, die Spitze definiert entweder einen Linienkontakt, einen Punktkontakt oder einen gezackten Kontakt.

4. Ein Drehwerkzeug für das Bohren in ein tierisches Nagel- und Klauen-Gewebesubstrat nach Anspruch 1, wobei die Spitze einen ersten Abschnitt definiert, der ein Material umfasst, das funktionsbereit ist, sich mit einer Oberfläche des Substrats durch Reibung zu verbinden, und einen zweiten Abschnitt, der funktionsbereit ist, sich mit der Oberfläche durch Eindringen über einen Linienkontakt, einen Punktkontakt oder einen gezackten Kontakt zu verbinden.

5. Ein Drehwerkzeug für das Bohren in ein tierisches Nagel- und Klauen-Gewebesubstrat nach Anspruch 1, wobei der Bohreinsatz ein Verbundteil umfasst, das ein erstes längliches Teil mit einem primären Schaft (403) an einem ersten Ende des länglichen Teils umfasst und eine Bohrschneide (302) an einem entgegengesetzten Ende des länglichen Teils, wobei ein sekundäres Schaftteil bereitgestellt wird mit einer kreisförmigen zylindrischen Symmetrie um eine Achse und eine längliche Öffnung koaxial innerhalb der besagten Achse, in die der primäre Schaft eingesetzt und daran befestigt werden kann, um damit einen Verbund-Bohreinsatz mit einem Schaftdurchmesser bereitzustellen, der größer ist als ein Schneiddurchmesser.

6. Ein Drehwerkzeug für das Bohren in ein tierisches Nagel- und Klauen-Gewebesubstrat nach einem der Ansprüche 1-5, wobei das elastische Teil (408) ein Presssitz um oder innerhalb entweder der Spannvorrichtung oder dem Bohreinsatz ist, funktionsbereit, um das jeweils entweder die Spannvorrichtung oder den Bohreinsatz zu greifen.

7. Ein Drehwerkzeug für das Bohren in ein tierisches Nagel- und Klauen-Gewebesubstrat nach einem der Ansprüche 1-6, wobei das elastische Teil (408) ein Spannfutter umfasst, das mit dem Schaft des Bohreinsatzes verbunden ist, das Spannfutter hat eine Elastizität, um sich funktionsbereit mit der Spannvorrichtung zu verbinden.

8. Ein Drehwerkzeug für das Bohren in ein tierisches Nagel- und Klauen-Gewebesubstrat nach Anspruch 7, wobei das elastische Teil (408) ein Spannfutter umfasst, das um den Schaft des Bohreinsatzes angeordnet ist und sich ausdehnende elastische Finger hat, die sich ausdehnenden Finger sind funktionsbereit, sich in ein Oberflächen-Element einzuklicken, um bei mindestens i) einer Rückhaltung des Bohreinsatzes und ii) einem Hinweis auf die Sicherung des Bohreinsatzes zu unterstützen.

9. Ein Drehwerkzeug für das Bohren in ein tierisches Nagel- und Klauen-Gewebesubstrat nach einem der Ansprüche 1-8, wobei das Werkzeug so angeordnet ist, dass die Rotation der Spannvorrichtung und des Bohreinsatzes nur auftreten kann, wenn sich die Spitze mit einem Substrat verbunden hat, wodurch sich der Bohreinsatz von der Spitze nach außen bewegen kann, so dass sich der Bohreinsatz mit dem Substrat verbinden und hineinbohren kann.

10. Ein Drehwerkzeug für das Bohren in ein tierisches Nagel- und Klauen-Gewebesubstrat nach einem der Ansprüche 1-9, wobei ein drehmomentsensitiver Motor funktionsbereit ist, den Bohreinsatz und die Spannvorrichtung drehbar anzutreiben.

11. Ein Drehwerkzeug für das Bohren in ein tierisches Nagel- und Klauen-Gewebesubstrat nach einem der Ansprüche 1-10, wobei der Motor in eine erste und eine zweite Rotationsrichtung um die besagte Achse betrieben werden kann.

12. Ein Drehwerkzeug für das Bohren in ein tierisches Nagel- und Klauen-Gewebesubstrat nach einem der Ansprüche 1-11, wobei die Ummantelungs-Spitze eine ringförmige Öffnungsspitze mit einem Substrat-Verbindungsteil bereitstellt.

13. Ein Drehwerkzeug für das Bohren in ein tierisches Nagel- und Klauen-Gewebesubstrat nach einem der Ansprüche 1-12, wobei die Öffnungsspitze ringförmig ist und einen drucksensitiven Sensor beinhaltet, wodurch eine Rotation bei Verbindung der ringförmigen Öffnungsspitze mit einer Oberfläche ermöglicht wird.

14. Ein Drehwerkzeug für das Bohren in ein tierisches Nagel- und Klauen-Gewebesubstrat nach einem der Ansprüche 1-13, wobei der Durchmesser des Bohreinsatzes und ein Durchmesser der ringförmigen Öffnungsspitze so ausgewählt werden, dass, wenn sie sich durch die Öffnung der Ummantelung erstrecken, der Bohreinsatz eng in die Öffnung der Ummantelung passt, wodurch er Unterstützung bietet und den Durchgang von Abrieb von einem Substrat und/oder den Durchgang einer zu reinigenden Flüssigkeit und/oder den Durchgang eines Medikaments zum Substrat ermöglicht.

15. Ein Drehwerkzeug für das Bohren in ein tierisches Nagel- und Klauen-Gewebesubstrat nach einem der Ansprüche 1-14, wobei die Ummantelung das Sammeln von Abrieb aus einem Substrat für Biopsiezwecke oder sonstiges ermöglicht.

## Revendications

1. Outil rotatif pour clou et griffe destiné au perçage à travers le substrat de tissus animaux des ongles et des griffes (400), comprenant un corps (405), un mandrin (411), un foret (302) et un capot (402), dans lequel ;
le corps fournit une fixation destinée au mandrin pour activer la rotation du mandrin autour d'un axe de rotation (Z), le mandrin étant fonctionnellement associé à un entraînement d'entrée rotatif ;
le foret est un élément allongé présentant une queue cylindrique, à une première extrémité proximale, un bord de coupe à une extrémité distale ;
le mandrin comprend une ouverture cylindrique circulairement coaxiale avec ledit axe de rotation et est utilisable pour accepter une queue (304) du foret, **caractérisé en ce qu'**il est fourni un élément résilient (408) utilisable pour aider à l'accouplement du foret du mandrin,
le capot (402) présentant une partie d'accouplement proximale (407) qui entre en prise avec le corps de l'outil et une extrémité distale qui définit un embout d'ouverture annulaire (406) autour de l'axe de rotation, l'ouverture du capot présentant un diamètre suffisant pour permettre le mouvement axial de l'extrémité distale du perçage à travers celui-ci, l'embout étant utilisable pour entrer en prise avec un substrat ; et,
dans lequel le corps n'est pas utilisable pour permettre le mouvement axial du mandrin et du foret par rapport au capot de façon à ce que le foret puisse s'étendre à travers l'embout.

2. Outil rotatif destiné au perçage à travers le substrat de tissus animaux des ongles et des griffes selon la revendication 1, dans lequel l'embout du capot (406) n'est pas utilisable pour entrer en prise avec le substrat au moyen de la friction, l'embout étant pourvu d'un composé en caoutchouc ou de type caoutchouc/polymère.

3. Outil rotatif destiné au perçage à travers le substrat de tissus animaux des ongles et des griffes selon la revendication 1, dans lequel l'embout du capot est utilisable pour entrer en prise physiquement avec le substrat au moyen de l'embout pénétrant dans la surface du substrat, l'embout définissant un élément parmi une ligne de contact, un point de contact ou un contact crénelé.

4. Outil rotatif destiné au perçage à travers le substrat de tissus animaux des ongles et des griffes selon la revendication 1, dans lequel l'embout définit une première section comprenant un matériau utilisable pour entrer en prise avec une surface du substrat par friction et une seconde section utilisable pour entrer en prise avec la surface par pénétration, au moyen d'une ligne de contact, d'un point de contact ou d'un contact crénelé.

5. Outil rotatif destiné au perçage à travers le substrat de tissus animaux des ongles et des griffes selon la revendication 1, dans lequel le foret comprend un élément composite comprenant un premier élément allongé présentant une queue principale (403) à une première extrémité de l'élément allongé et un outil de coupe (302) à une extrémité opposée de l'élément allongé, dans lequel un élément de queue secondaire est fourni présentant une symétrie cylindrique circulaire autour d'un axe et une ouverture allongée coaxiale à l'intérieur dudit axe, dans lequel la queue primaire peut être insérée et fixée à celle-ci, ce qui permet de fournir un foret composite comportant un diamètre de queue supérieur à un diamètre de coupe.

6. Outil rotatif destiné au perçage à travers le substrat de tissus animaux durs selon l'une quelconque des revendications 1 à 5, dans lequel l'élément résilient (408) est un ajustement serré autour ou à l'intérieur d'un élément parmi le mandrin et le foret et est utilisable pour serrer l'autre élément parmi le mandrin et le foret.

7. Outil rotatif destiné au perçage à travers le substrat de tissus animaux durs selon l'une quelconque des revendications 1 à 6, dans lequel l'élément résilient (408) comprend un collet, associé à la queue du foret, le collet présentant une résilience, ce qui lui permet d'entrer en prise fonctionnellement avec le mandrin.

8. Outil rotatif destiné au perçage à travers le substrat de tissus animaux durs selon la revendication 7, dans lequel l'élément résilient (408) comprend un collet, disposé autour d'une queue du foret et présente des doigts résilients s'étendant, lesquels doigts résilients s'étendant sont utilisables pour se clipser dans une caractéristique de surface du mandrin, ce qui permet d'aider à au moins un parmi i) la rétention du foret et, ii) l'indication de l'arrimage du foret.

9. Outil rotatif destiné au perçage à travers le substrat de tissus animaux durs selon l'une quelconque des revendications 1 à 8, dans lequel l'outil est disposé de sorte que la rotation du mandrin et du foret ne peut se produire qu'après l'entrée en prise de l'embout avec un substrat, après quoi le foret est autorisé à se déplacer vers l'extérieur de l'embout, de sorte que, le foret peut entrer en prise et percer dans le substrat.

10. Outil rotatif destiné au perçage à travers le substrat de tissus animaux durs selon l'une quelconque des revendications 1 à 9, dans lequel un couple moteur raisonnable est utilisable pour entraîner en rotation le foret et le mandrin.

11. Outil rotatif destiné au perçage à travers le substrat de tissus animaux durs selon l'une quelconque des revendications 1 à 10, dans lequel le moteur est utilisable dans de premier et second sens de rotation autour dudit axe.

12. Outil rotatif destiné au perçage à travers le substrat de tissus animaux durs selon l'une quelconque des revendications 1 à 11, dans lequel l'apex du capot fournit un embout d'ouverture annulaire comportant une partie d'entrée en prise du substrat.

13. Outil rotatif destiné au perçage à travers le substrat de tissus animaux durs selon l'une quelconque des revendications 1 à 12, dans lequel l'embout d'ouverture est annulaire et comprend un capteur sensible à la pression, ce qui permet d'activer la rotation sur l'entrée en prise de l'embout d'ouverture annulaire avec une surface.

14. Outil rotatif destiné au perçage à travers le substrat de tissus animaux durs selon l'une quelconque des revendications 1 à 13, dans lequel le diamètre du foret et d'un diamètre de l'embout d'ouverture annulaire sont choisis de sorte que, lorsqu'il est étendu à travers l'ouverture du capot, le foret est ajusté serré par rapport à l'ouverture du capot, ce qui permet de fournir un support et d'ouvrir le passage des copeaux d'un substrat et/ou le passage d'un fluide de nettoyage et/ou le passage d'un médicament pour le substrat.

15. Outil rotatif destiné au perçage à travers le substrat de tissus animaux durs selon l'une quelconque des revendications 1 à 14, dans lequel le capot permet la collecte des copeaux à partir d'un substrat à des fins de biopsie ou autrement.
